# EUROPEAN PATENT APPLICATION

(11) **EP 2 500 002 A1**
(43) Date of publication of application: **19.09.2012**
(21) Application number: 11158598.0
(22) Date of filing: 17.03.2011
(51) Int. Cl.: A61F 9/00

(54) **Apparatus for intraocular injection**

(71) Applicant: Sanofi-Aventis Deutschland GmbH, 65929 Frankfurt (DE)
(72) Inventor: The designation of the inventor has not yet been filed
(74) Representative: KEIL & SCHAAFHAUSEN Patentanwälte

(57) **Abstract**

Described is an apparatus for intraocular injection comprising a body adapted to accommodate an injection device and a displacement device coupled to a distal portion of the body. The displacement device comprises a first portion and a second portion connected to the first portion. Axial movement of the first portion within the body in a distal direction causes the second portion to displace a superficial layer of an eye relative to an underlying layer of the eye.

## Description

The present invention relates to an apparatus for intraocular injection and a corresponding method. An intraocular injection is used to treat eyes, such as eyes of mammals having eye disorders or diseases.

### Background

A number of vision-threatening disorders or diseases of the eye need to deliver a drug (medicament or proteins or the like) by intraocular delivery (more specifically intravitreal delivery), especially when it is useful to deliver high concentrations of drugs. One such technique for intraocular delivery is accomplished by intraocular injection of the drug or capsules containing the drug directly into the vitreous body or by locating a device or capsule containing the drug in the vitreous with a syringe. Such an operation is used in particular for injection of compositions in the vitreous body of the eye in order to treat diseases affecting the retina or choroid, or ciliary body or the lens.

After delivery of drugs to the interior of the eye, such as the vitreous body, it is desirable that a point of entry of any drug delivery device closes and heals or seals as quickly and completely as possible after withdrawal of the drug delivery device. Sealing prevents reflux of the delivered drug, reduces internal eye pressure, heals the eye tissue affected (e.g. sclera), and prevents infections and other complications.

An apparatus for intraocular injection is known from documents WO 2008/084063 A1 and WO 2008/084064 A1. These documents describe a technique wherein the superficial layer of the eye (conjunctiva) is urged to slide over the underlying layer (sclera) by a flexible leg of a resilient member during a downward movement of the whole apparatus into the direction of the eye so that the layers are shifted one relative to the other prior to the needle penetrating into the eye. When the injection apparatus and hence the resilient member are removed from the eye, the superficial layer, i.e. the conjunctiva, slides over the underlying layer (sclera) back to its initial position.

The known apparatus is constructed in the way that the flexible leg is the first portion of the apparatus to come into contact with the eye. Thus, if the leg does not grip the superficial layer of the eye or simply flexes without causing displacement of the superficial layer, the desired displacement of the superficial layer over the underlying layer will not be achieved. Further, during downward movement of the known apparatus, the placement of the apparatus may be imprecise and therefore the point of insertion of the needle may be incorrect. However, it is important to exactly find the right position for puncturing the eye in order to avoid damaging structures located in front or in the rear of the vitreous body. The known apparatus may tend to slide away from the desired point of insertion.

It is therefore an object of the present invention to provide an apparatus for intraocular injection which could precisely be positioned in a desired zone of the eye and would allow for displacement of the superficial layer of the eye relative to the underlying layer prior to drug delivery and return of the superficial layer to its original position after drug delivery to allow for, e.g., occlusion of the point of entry of the drug delivery device. Accordingly, a corresponding method is presented.

### Summary of the Invention

This problem is solved with an apparatus having the features of claim 1.

In an exemplary embodiment, an apparatus for intraocular injection comprises a body adapted to accommodate an injection device and a displacement device coupled to a distal portion of the body. The displacement device comprises a first portion and a second portion connected to the first portion. Axial movement of the first portion within the body in a distal direction causes the second portion to displace a superficial layer of an eye relative to an underlying layer of the eye. The second portion may be adapted to perform a linear and/or rotational movement in order to displace the superficial layer of the eye over the underlying layer.

The apparatus may further comprise an apparatus placement foot coupled to the distal portion of the body. The apparatus placement foot may include a positioning component for aligning the apparatus placement foot on the eye.

The first portion may be connected to the second portion via a spring, which biases the injection device in a retracted position within the body.

The second portion may comprise an arm having a proximal end coupled to the second portion and a distal end coupled to a manipulating foot. Distal surfaces of the manipulating foot and the placement foot may be in a same plane.

In exemplary embodiments, the second portion may comprise a rotatable manipulating rod, at least two hinged gripping legs, and/or a displacement limiting mechanism. Axial movement of the first portion in a proximal direction may cause the second portion to at least one of (i) return the superficial layer to a starting position and (ii) release the superficial layer.

The injection device may comprise a needle, and the needle may be inserted into the eye after the second portion has displaced the superficial layer.

In another exemplary embodiment, the invention includes a conjunctiva displacement device comprising a first portion adapted to engage an injection device and moveable in a first plane and a second portion coupled to the first portion. The second portion may be adapted to displace a superficial layer of an eye in a second plane based on movement of the first portion in the first plane.

These as well as other advantages of various aspects of the present invention will become apparent to those of ordinary skill in the art by reading the following detailed description, with appropriate reference to the accompanying drawings.

### Brief Description of the Drawings

Exemplary embodiments are described herein with reference to the schematic drawings in which:
- Fig. 1: illustrates an exemplary embodiment of an apparatus for intraocular injection in a general cross section prior to displacement of a conjunctiva and delivery of injection;
- Fig. 2: shows the cross section of Fig. 1 after conjunctiva displacement and needle insertion;
- Fig. 3: illustrates a perspective view of a displacement device of the exemplary embodiment shown in Figures 1 and 2;
- Figures 4a and 4b: show a detailed view of Figures 1 and 2, respectively;
- Figures 5 and 6: show a perspective view of a distal portion of an apparatus according to the exemplary embodiment shown in Figures 1 to 4b prior (Fig. 5) and during (Fig. 6) injection of the needle;
- Fig. 7: shows a sectional view of another exemplary embodiment of an apparatus for intraocular injection in a starting position in a perspective view (left) and in a front view (right);
- Fig. 8: illustrates the exemplary embodiment and views of Fig. 7 at the beginning of displacing of conjunctiva over sclera;
- Fig. 9: depicts the exemplary embodiment and views of Figures 7 and 8 in a moment in which the needle punctures the eye;
- Fig. 10: shows a perspective view of a displacement device of the exemplary embodiment shown in Figures 7 to 9;
- Fig. 11: illustrates a sectional view of another exemplary embodiment of an apparatus for intraocular injection prior to injection;
- Fig. 12: shows the sectional view of the exemplary embodiment of Fig. 11 in a moment in which the needle of the syringe punctures the eye;
- Fig. 13: depicts a detailed view of Fig. 11;
- Fig. 14: depicts a detailed view of Fig. 12; and
- Fig. 15: illustrates a displacement device of the exemplary embodiment shown in Figures 11 to 14 in a perspective view.

### Detailed Description

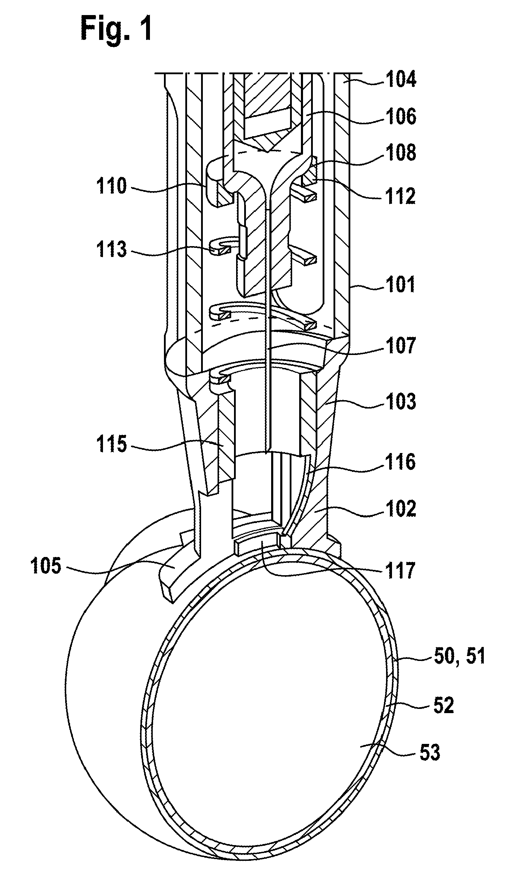

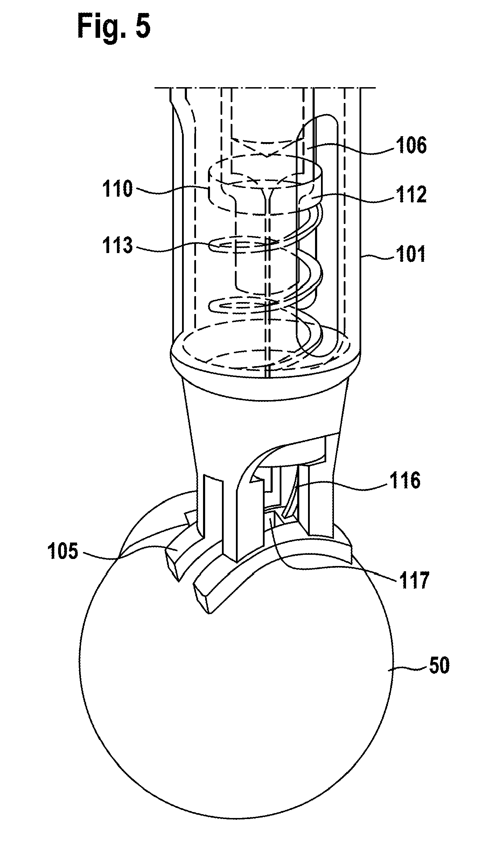

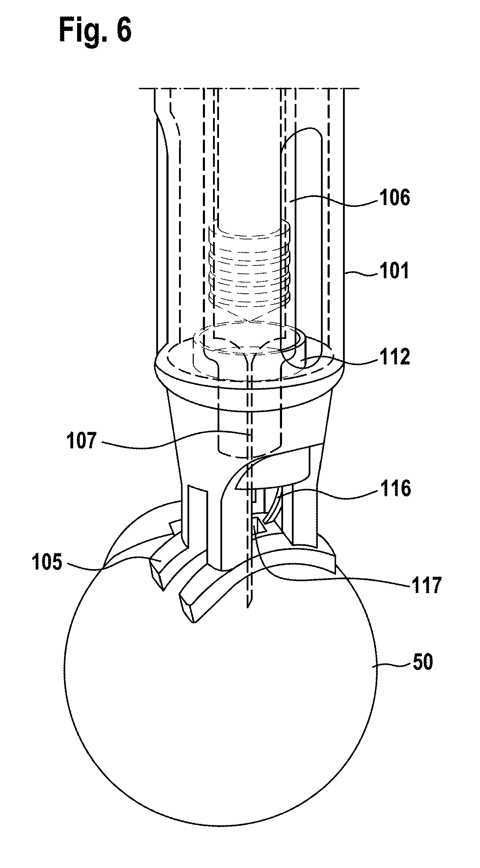

Figures 1 to 6 illustrate an exemplary embodiment of an apparatus for intraocular injection comprising a body 101. The body 101, which may generally be formed as a hollow tube to accommodate an injection device, e.g., a syringe 106 or a cartridge and needle, comprises a distal section 102, a middle section 103 and a proximal section 104. The distal section 102 of the body 101 includes an apparatus placement foot 105 which may rest on an external surface of an eye 50 as depicted in Figures 1, 2, 4a, 4b, 5, and 6. In an exemplary embodiment, the foot 105 may include a positioning component on a lateral portion thereof which facilitates positioning of the apparatus for injection. For example, the positioning component may be a circular portion which is meant to be aligned with a circumference of a cornea of the eye 50. As shown in the exemplary embodiment in Figure 1, the positioning component may be arranged so that, when properly aligned with the cornea, the apparatus is positioned to deliver an injection through a conjunctiva 51 and sclera 52 and into a posterior portion of the eye 50 and thereby ensures that a needle 107 of the syringe 106 does not pierce the limbus or the lens.

The body 101 is sized and shaped to receive the syringe 106 therein. The body 101 and the syringe 106 may be separate components or formed as a single device (e.g., a user does not have access to the syringe 106). A neck 108 of the syringe 106 is supported by a proximal end of a conjunctiva displacement device 110 disposed at a distal end of the body 101.

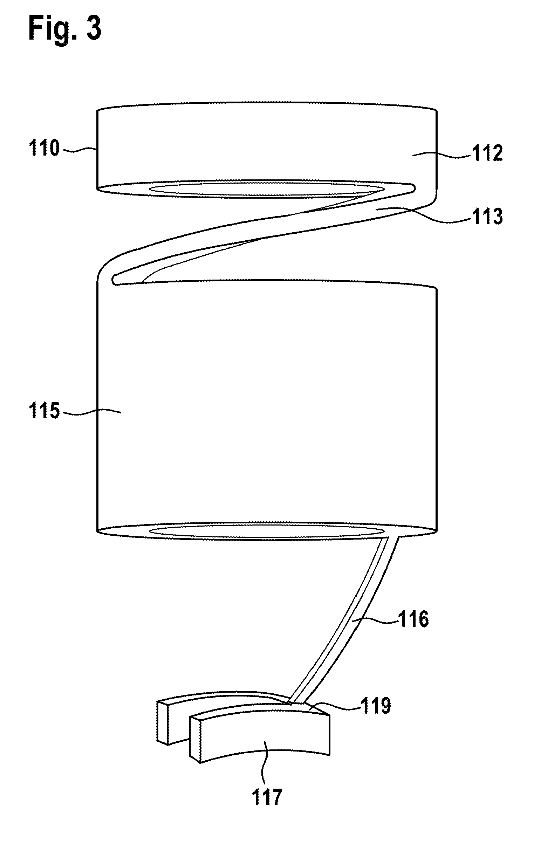

In the exemplary embodiment as shown in Figure 3, the conjunctiva displacement device 110 comprises a proximal end forming an annular supporting ring 112 which abuts the neck 108 of the syringe 106 and a second ring 115, distal to the supporting ring 112. The supporting ring 112 and the second ring 115 are coupled together via a coil spring 113. A distal end of the second ring 115 is coupled to a proximal end of an arm 116. A distal end of the arm 116 is coupled to a manipulating foot 117, which in this exemplary embodiment is depicted as being U-shaped. The displacement device 110 may be produced as one piece, for example by molding, and be made from various materials alone (e.g., plastic) or in combination (e.g., metal and plastic).

In an exemplary embodiment, the proximal end of the arm 116 may be hingedly connected to the second ring 115, and the distal end of the arm 116 may be hingedly connected to the manipulating foot 117. In another exemplary embodiment, the proximal end of the arm 116 may be fixed to the second ring 115, and the distal end of the arm 116 may be fixed to the manipulating foot 117. In the latter embodiment, the arm 116 may be elastically deformable, having a spring-like effect.

In the exemplary embodiment shown in Fig. 1 to 6, the foot 105 surrounds the manipulating foot 117 partially. Alternatively, it is also possible that foot 105 surrounds manipulating foot 117 fully.

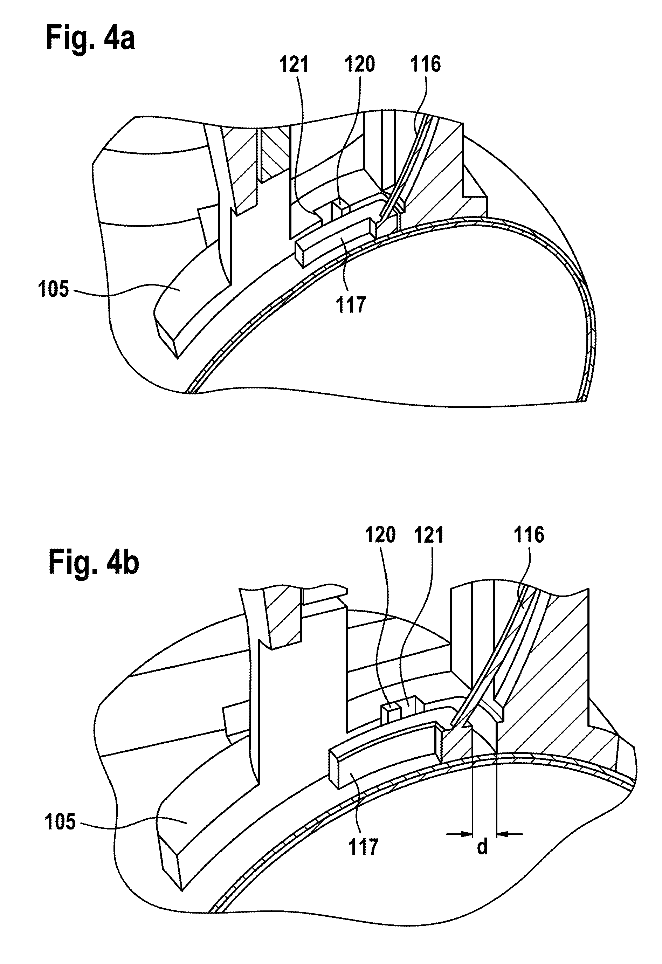

In an exemplary embodiment, the arm 116 is connected to the manipulating foot 117 at a central section 119 of thereof. As shown in the exemplary embodiments depicted in Figures 4a and 4b, the manipulating foot 117 includes a displacement limiting mechanism comprising a cam 120 moveable within a recess 121 formed in the foot 105. A length of the recess 121 may be selected based on a desired displacement of the manipulating foot 117 in a first direction. The foot 105 may also have a stop formed behind the central section 119 which may limit movement of the manipulating foot 117 in a second direction.

In an exemplary embodiment, the apparatus may be utilized to administer a drug or the like into an eye, e.g. the vitreous body. Prior to use, the needle 107 of the syringe 106 may be contained within the body 101, e.g., to prevent injury, and a distal opening of the body 101 and/or the foot 105 may be covered with a film to maintain sterility of the needle 107. The needle 107 may be covered with a cap (not shown).

In the exemplary embodiment, the displacement device 110 is accommodated within the body 101 at a distal end thereof. During medical treatment using the inventive apparatus, at first a physician moves the eye lids of the patient apart using an eye lid retractor. The foot 105 may then be used to align with the cornea to ensure that the injection site will not pierce the cornea, lens or limbus, but be directed into the vitreous. Those of skill in the art will understand that the foot 105 (or a portion thereof) may be made from a transparent material such that alignment with a periphery of the cornea may be facilitated. Preferably, distal surfaces of the foot 105 and the manipulating foot 117 are in the same plane such that when the foot 105 is positioned on the eye, the manipulating foot 117 is also in contact with the eye.

When the apparatus has been properly placed on the eye 50, the physician may depress a plunger or similar depressable element coupled to the body 101 and/or the syringe 106 which advances the syringe 106 distally within the body 101 towards the injection site. As the syringe 106 moves distally within the body 101, the neck 108 abuts the ring 112 and urges it distally, compressing the spring 113 and urging the second ring 115 distally.

In an exemplary embodiment, the distal movement of the second ring 115 causes the arm 116 to bend, pushing the manipulating foot 117 in the first direction, e.g., laterally and circumferentially across the eye. Because the manipulating foot 117 is in contact with the conjunctiva 51, movement of the manipulating foot 117 will result in corresponding movement of the conjunctiva 51, displacing the conjunctiva 51 relative to the sclera 52, prior to insertion of the needle 107. In an exemplary embodiment, the displacement of the conjunctiva 51 in the first direction may be limited to a pre-determined distance d (see Fig. 4b). The pre-determined distance d may be limited by the length of the recess 121.

In another exemplary embodiment, the arm 116 may be disposed at an angle with respect to the second ring 115 and the manipulating foot 117 such that a distal force applied to the arm 116 causes, additionally or alternatively to the bending of the arm 116, the angle with the second ring 115 to decrease and/or the angle with the foot 117 to increase, respectively, turning the respective hinges, and pushing the foot 117 (and the conjunctiva 51) in the first direction.

Those of skill in the art will understand that an underside of the foot 117 (e.g., a surface of the foot 117 which contacts the conjunctiva 51) may include a frictional layer or other means for gripping, without injury, the conjunctiva 51. It is preferred that movement of the foot 117 does not change a position of the sclera 52.

When the foot 117 is prevented from further movement (by interaction of the cam 120 within the recess 121), further distal movement of the syringe 106 within the body 101 compresses the spring 113. The displacement of the conjunctiva 51 is completed before the needle 107 punctures the eye 50. At this time the above explained first or displacement step is finished.

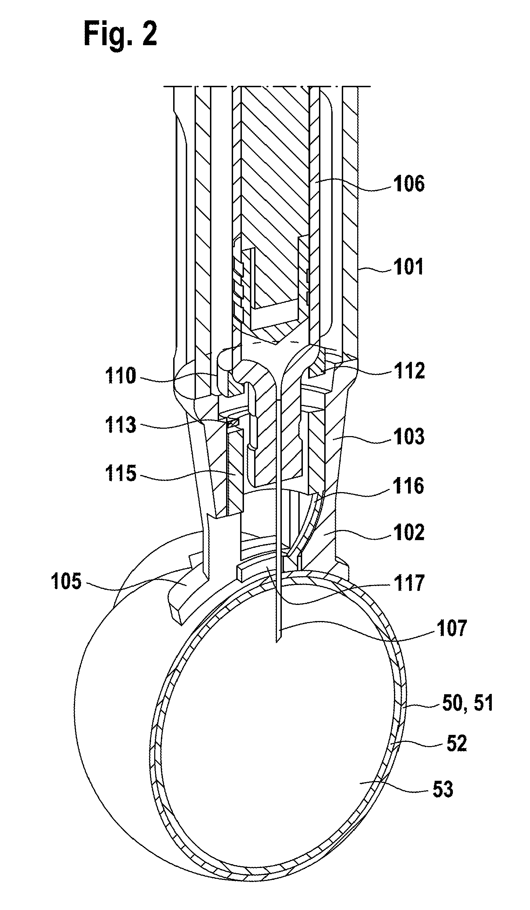

The situation where needle 107 punctures the eye 50 is illustrated in Fig. 2. During puncturing, the needle 107 first penetrates the displaced conjunctiva 51, then the sclera 52 and after that it penetrates into the vitreous body 53 of the eye 50. In this position, the syringe 106 may be prevented from further axial movement within the body 101, and distally-directed force on the plunger of the syringe 106 causes the drug or the like contained within the syringe 106 to be administered into the vitreous body 53 (intravitreal injection). Examples of such a drug are steroids or monoclonal antibodies used to treat macular degeneration. Those of skill in the art will understand that various medicaments and/or therapeutic substances and/or implantable devices may be administered using the apparatus.

In an exemplary embodiment, after dispensing the drug, the syringe 106 is returned to its starting position and apparatus is removed from the eye 50. The spring 113 may force the syringe 106 in a proximal direction, allowing the foot 117 to move in the second direction and the arm 116 to return to its starting position. Movement of the foot 117 in the second direction (and, in conjunction with an elastic nature of the conjunctiva 51) returns the conjunctiva 51 to its original position. The central section 119 of the displacement device 110 may limit movement of the foot 117 in the second direction as it returns to its starting position. When the syringe 106 returns to its starting position, it is preferable that the needle 107 is retracted within the body 101 or displacement device 110 such that a tip of the needle 107 is not exposed, thus preventing a needlestick injury. In other exemplary embodiments, a manual or automatic needle shield may be utilized to cover the needle 107 (or distal opening of the body 101 or the displacement device 110) after use. Similarly, a locking mechanism (not shown) may be utilized to prevent the syringe 106 from moving axially within the body 101 after the injection has been administered and the syringe 106 has returned to its original position

By displacing the conjunctiva 51 relative to the sclera 52 during the injection procedure, a punctured region (orifice) of the conjunctiva 51 is offset to the punctured region (orifice) of sclera 52, e.g., by the distance d, when the conjunctiva 51 is returned to its original position. Hence, the conjunctiva 51 seals the orifice of the sclera 52, which may, for example, prevent reflux of the delivered drug, reduce the effects of the procedure on internal eye pressure, assist with the healing of the eye 50, and reduces the risk of infection.

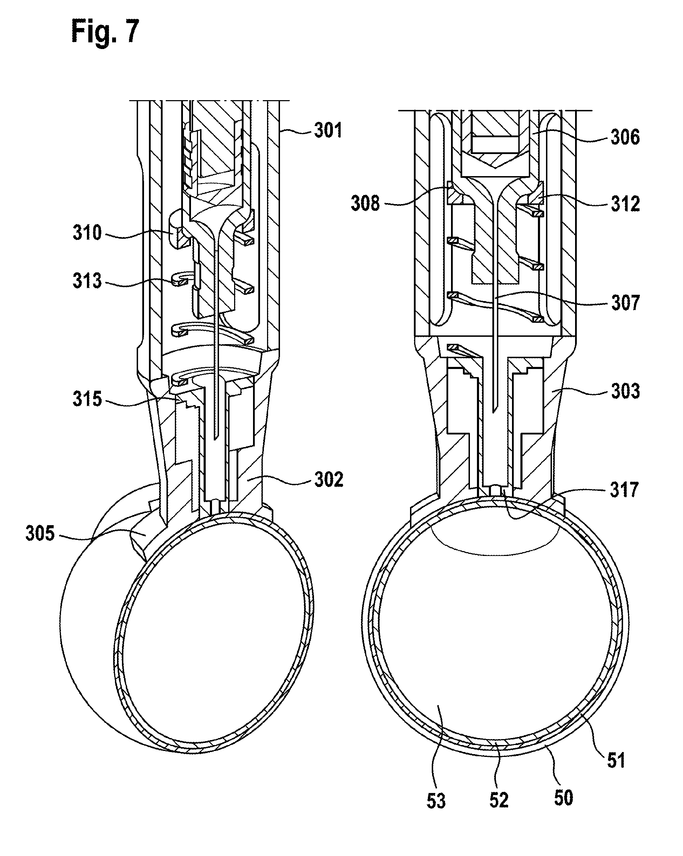

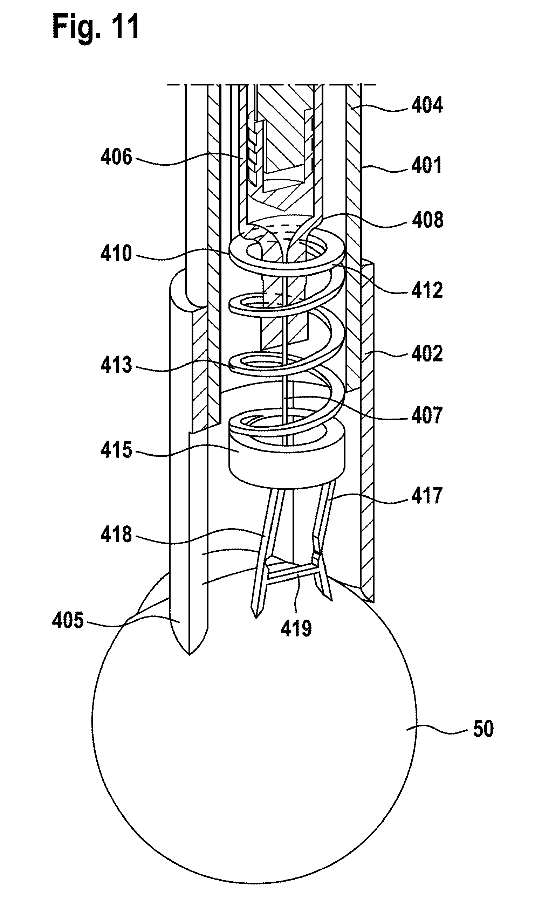

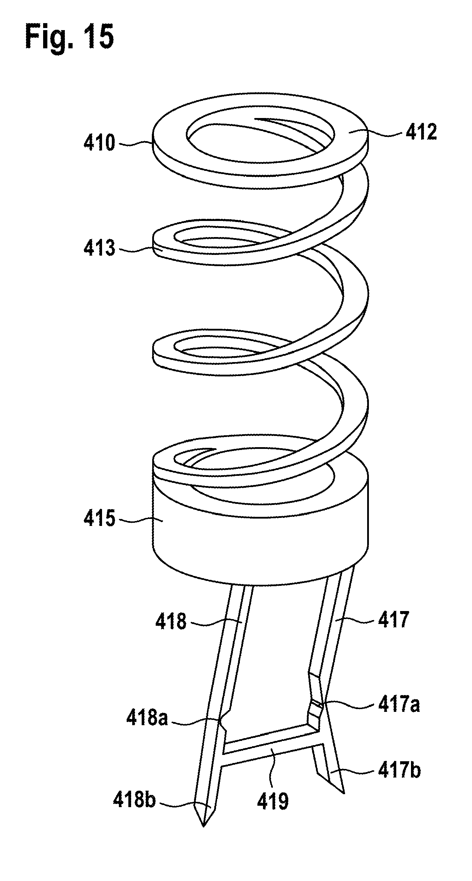

Figures 7 to 10 show another exemplary embodiment of an apparatus for intraocular injection. The differences between this exemplary embodiment and the exemplary embodiment described above can derived from the following explanations, wherein the reference numbers containing the same digits at the last position and next to last position of the number refer to the same elements as in the exemplary embodiment described above if not otherwise explained below. The same applies to another exemplary embodiment depicted in Figures 11 to 15.

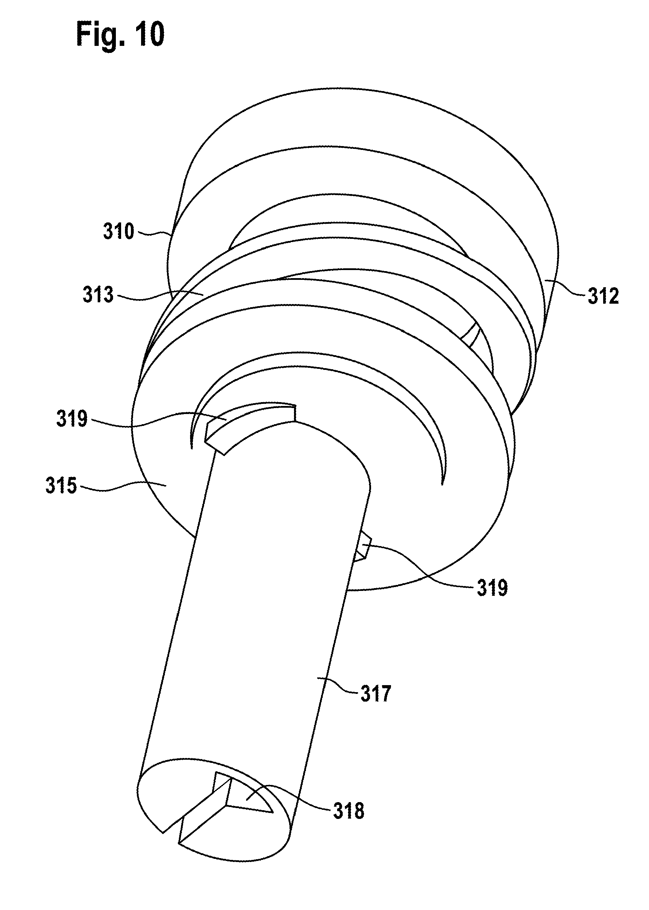

As shown in Figure 10, in an exemplary embodiment, a conjunctiva displacement device 310 includes a supporting ring 312, a spring 313, a circular plate 315 and a manipulating rod 317. These elements are connected to each other in the mentioned order as it is shown in Fig. 10. The displacement device 310 may be formed as one integral (for example molded) piece or may be composed of several pieces.

As shown in the exemplary embodiment in Figure 10, the manipulating rod 317 is formed basically as a rod having a channel 318 through which the needle 307 may pass to deliver an injection to the eye 50. The plate 315 has a respective opening (not shown) aligned with the channel 318. The manipulating rod 317 is attached to (e.g., formed integrally with) the plate 315, extending distally therefrom.

In this exemplary embodiment, the device 310 includes a displacement limiting mechanism comprising a distal surface of the plate 315 includes one or more helical cams 319, and a proximal surface of a distal end of the body 301 comprises one or more corresponding helical cams (not shown) at its inner surface of the middle section 303. The supporting ring 312 forms the guiding portion of the displacement device 310 and the manipulating rod 317 is the displacement portion as described previously.

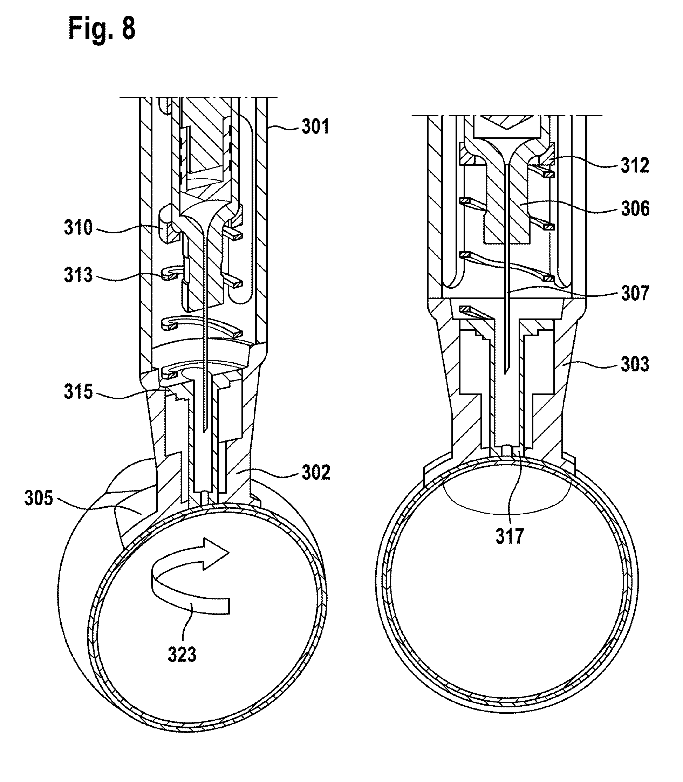

In the exemplary embodiment depicted in Fig. 7, the apparatus placement foot 305 is aligned with the cornea to position the apparatus for an injection. When properly aligned, the foot 317 and a distal end of the manipulating rod 317 rest on the conjunctiva 51 of the eye 50 at a pre-determined position, for example the limbus region. Now, in the first or displacement step, the syringe 306 is moved axially in a distal direction within the body 301 from a starting position and contacts the supporting ring 312. The syringe 306 distally displaces the supporting ring 312 of the displacement device 310 relative to the body 301. When the cams 319 on the plate 315 engage the corresponding cams on the body 301, further distal force causes the manipulating rod 317 to rotate in a first rotational direction. This rotation (marked in Fig. 8 by arrow 323) causes the portion of the conjunctiva 51 trapped beneath the manipulating rod 317 to be displaced relative to the sclera 52 (see Fig. 8). The rotation continues until the cams 319 abut a non-cammed proximal surface of the body 301 or a respective end face of the corresponding cams of the body 301. Those of skill in the art will understand that the length and angle of the cams 319 determine the maximum rotation and angular velocity of rotation of the manipulating rod 317.

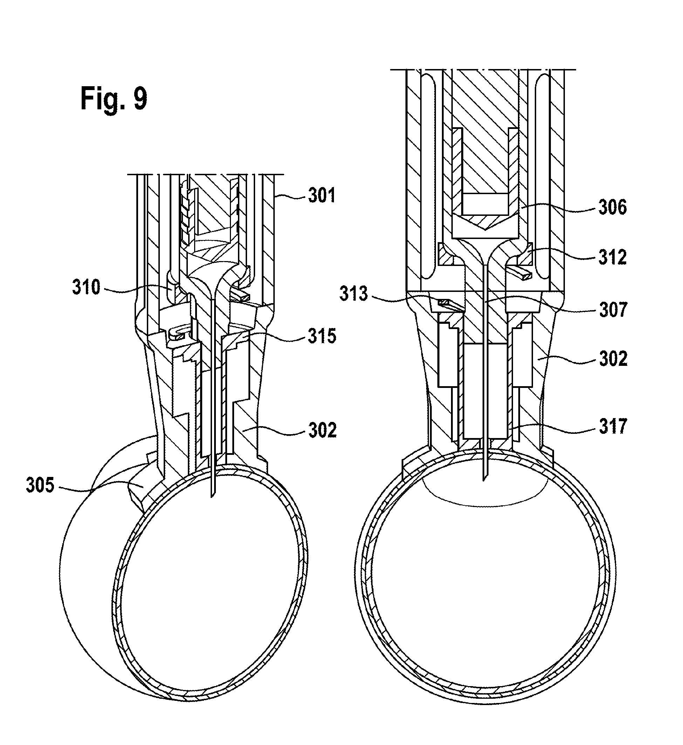

Subsequent distal movement of the syringe 306 is accommodated by the spring 313 and leads the needle 307 to puncture the conjunctiva 51, sclera 52 and the vitreous body 53 of eye 50. When the spring 313 cannot be compressed further, distally-directed pressure applied to a plunger on the syringe 306 causes the drug to be administered (ref. Fig. 9). By displacing the conjunctiva 51 relative to the sclera 52 during the injection procedure, a punctured region (orifice) of the conjunctiva 51 is offset to the punctured region (orifice) of sclera 52 when the conjunctiva 51 is returned to its original position. Hence, the conjunctiva 51 seals the orifice of the sclera 52, which may, for example, prevent reflux of the delivered drug, reduce the effects of the procedure on internal eye pressure, assist with the healing of the eye 50, and reduces the risk of infection.

In an exemplary embodiment, after dispensing the drug, the syringe 306 is returned to its starting position and apparatus is removed from the eye 50. The spring 313 may force the syringe 306 in a proximal direction, allowing the manipulating rod 318 to rotate in a second rotational direction back to its starting position. Rotation of the manipulating rod 318 in the second rotational direction (and, in conjunction with an elastic nature of the conjunctiva 51) returns the conjunctiva 51 to its original position. When the syringe 306 returns to its starting position, it is preferable that the needle 307 is retracted within the body 301 or displacement device 310 such that a tip of the needle 307 is not exposed, thus preventing a needlestick injury. In other exemplary embodiments, a manual or automatic needle shield may be utilized to cover the needle 307 (or distal opening of the body 301 or the displacement device 310) after use. Similarly, a locking mechanism (not shown) may be utilized to prevent the syringe 306 from moving axially within the body 301 after the injection has been administered and the syringe 306 has returned to its original position.

Another exemplary embodiment of an apparatus for intraocular injection is depicted in Figures 11 to 15. In this exemplary embodiment, the conjunctiva displacement device 410 consists of an annular supporting ring 408 forming a proximal guiding portion, a spring 413, and a second annular ring 415. Deformable legs 417, 418 form a distal displacement portion of the displacement device 410. The legs 417,418 are attached to the distal end of second ring 415 and connected by a cross rod 419. In an exemplary embodiment, each leg 417, 418 includes one or more grooves or hinges 417a and 418a formed above the cross rod 419. The cross rod 419 aligns the legs 417, 418 ensuring that they move in the same plane. The legs 417, 418 may also utilize the hinges 417a, 418a as a displacement limiting mechanism, as described below.

In this exemplary embodiment, the body 401 may comprise an outer sleeve 402 and an inner sleeve 404. The outer sleeve 402 fits telescopically on the inner sleeve 402 and is movable between first (retracted) and second (extended) positions. When the apparatus is being positioned for an injection, the outer sleeve 402 may be in the retracted position to allow the physician to visualize the placement and alignment of the apparatus and the potential injection site.

When the injection is being administered or prior thereto (but after initial placement of the apparatus on the eye 50), the outer sleeve 402 may be deployed to the extended position to maintain sterility of the injection site. Those of skill in the art will understand that the outer sleeve 402 may be utilized in any of the embodiments described herein.

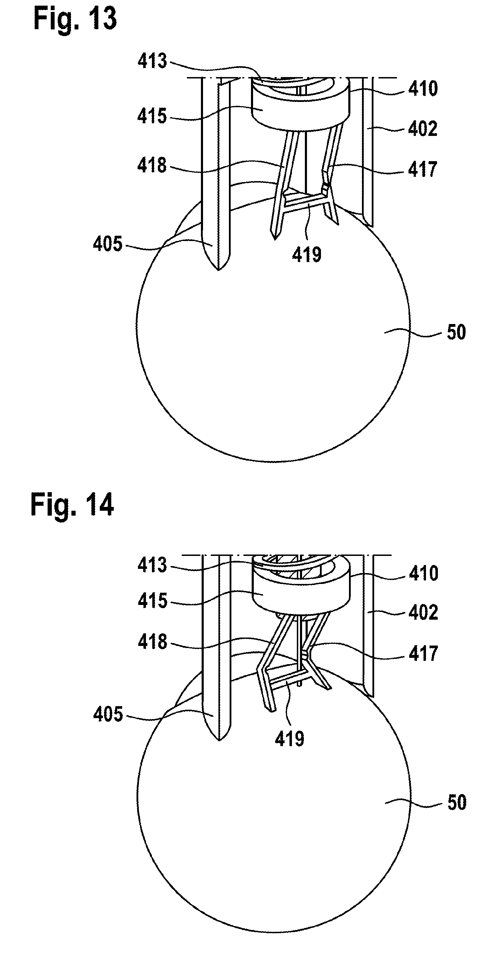

At the starting position shown in Figures 11 and 13, the apparatus is placed against the eye 50. In this exemplary embodiment, an apparatus placement foot 405 is formed from a distal end of the outer sleeve 402. In another exemplary embodiment, the placement foot 405 may be formed on a distal end of the inner sleeve 404. In the starting position, the legs 417, 418 may be in contact with the conjunctiva 51 but are not displacing it. The distal ends of legs 417, 418 form tips 417b, 418b, respectively, which may partly penetrate into the conjunctiva 51 in order to ensure good grip or may include some other mechanism (rubber/polymeric cuffs) for ensuring a frictional hold on the surface of the conjunctiva 51.

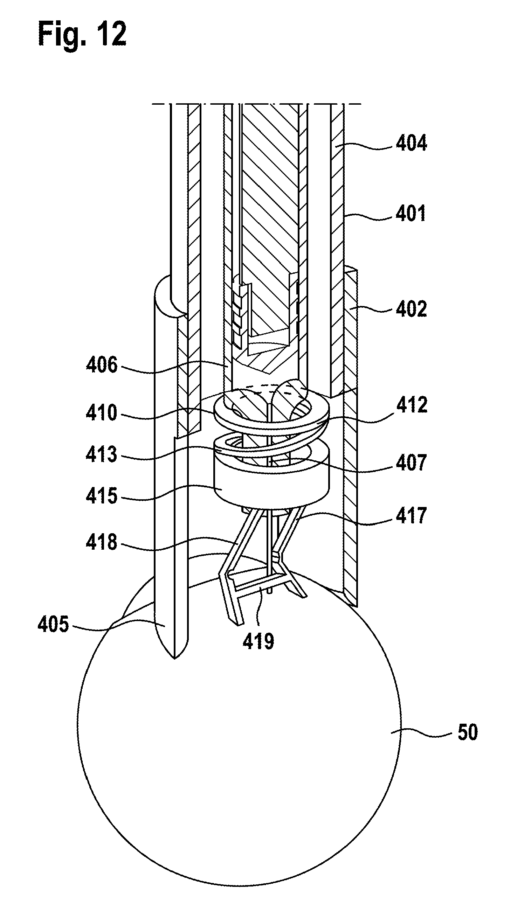

When the syringe 406 is advanced axially in the distal direction, the syringe neck 408 applies a distally-directed force on the first annular ring 412. The force is transmitted to the spring 413, which causes distal displacement of the second ring 415 and causes the legs 417 and 418 to buckle at the position of the grooves or hinges 417a, 418a. Thereby, the conjunctiva 51 is displaced laterally and circumferentially relative to the sclera 52. After the displacement of the conjunctiva 51, a force required to further buckle the legs 417, 418 is greater than the force required to compress the spring 413. Thus, the syringe 406 is further advanced distally, compressing the spring 413 and allowing the needle 407 to puncture the displaced conjunctiva 51, the sclera 52 and the vitreous body 53 (see Figures 12 and 14).

After administration of the injection, the force of the spring 413 urges the syringe 406 proximally, and the legs 417, 418 relax back to their original position, concurrently dragging the conjunctiva 51 (and allowing for the elasticity of the conjunctiva 51 to return it) back to its original position so that the conjunctiva 51 seals the orifice of the sclera 52.

By displacing the conjunctiva 51 relative to the sclera 52 during the injection procedure, a punctured region (orifice) of the conjunctiva 51 is offset to the punctured region (orifice) of sclera 52 when the conjunctiva 51 is returned to its original position. Hence, the conjunctiva 51 seals the orifice of the sclera 52, which may, for example, prevent reflux of the delivered drug, reduce the effects of the procedure on internal eye pressure, assist with the healing of the eye 50, and reduces the risk of infection.

When the syringe 406 returns to its starting position, it is preferable that the needle 407 is retracted within the body 401 or displacement device 410 such that a tip of the needle 407 is not exposed, thus preventing a needlestick injury. In other exemplary embodiments, a manual or automatic needle shield may be utilized to cover the needle 407 (or distal opening of the body 401 or the displacement device 410) after use. Similarly, a locking mechanism (not shown) may be utilized to prevent the syringe 406 from moving axially within the body 401 after the injection has been administered and the syringe 406 has returned to its original position.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### Reference numbers:

- 50: eye
- 51: conjunctiva
- 52: sclera
- 53: vitreous body
- 101,301,401: body
- 102, 302: distal section
- 103, 303: middle section
- 104, 304: proximal section
- 105, 305, 405: apparatus placement foot
- 106, 306, 406: syringe
- 107, 307: needle
- 108, 308, 408: neck of syringe
- 110, 310, 410: displacement device
- 112, 312, 412: supporting ring
- 113, 313, 413: coil spring
- 115, 415: second ring
- 116: arm
- 117: manipulating foot
- 119: central section of manipulating foot 117
- 120: cam
- 121: recess
- 317: manipulating rod
- 318: cutout
- 319: helical cam
- 323: arrow
- 402: outer sleeve
- 404: inner sleeve
- 417,418: leg
- 417a, 418a: hinge or groove
- 417b, 418b: tip
- 419: cross rod
- d: displacement distance of manipulating foot 107

## Claims

1. An apparatus for intraocular injection comprising
a body adapted to accommodate an injection device; and
a displacement device coupled to a distal portion of the body, wherein the displacement device comprises a first portion axially movable within the body and a second portion adapted to contact a superficial layer of an eye, wherein axial movement of the first portion within the body in a distal direction causes the second portion to displace the superficial layer of the eye relative to an underlying layer of the eye.

2. The apparatus according to claim 1 wherein the second portion is adapted to perform a linear and/or rotational movement in order to displace the superficial layer of the eye over the underlying layer.

3. The apparatus according to any of the preceding claims wherein the apparatus further comprises an apparatus placement foot coupled to the distal portion of the body.

4. The apparatus according to claim 2, wherein the apparatus placement foot includes a positioning component for aligning the apparatus placement foot on the eye.

5. The apparatus according to any of the preceding claims wherein the first portion is connected to the second portion via a spring.

6. The apparatus according to claim 5 wherein the spring biases the injection device in a retracted position within the body.

7. The apparatus according to any of the preceding claims wherein the second portion comprises an arm having a proximal end coupled to the second portion and a distal end coupled to a manipulating foot.

8. The apparatus according to claim 7 wherein distal surfaces of the manipulating foot and the placement foot are in a same plane.

9. The apparatus according to any of the claims 1 to 6 wherein the second portion comprises a rotatable manipulating rod.

10. The apparatus according to any of the claims 1 to 6 wherein the second portion comprises at least two hinged gripping legs.

11. The apparatus according to any of the preceding claims wherein the second portion comprises a displacement limiting mechanism.

12. The apparatus according to any of the preceding claims wherein axial movement of the first portion in a proximal direction causes the second portion to at least one of (i) return the superficial layer to a starting position and (ii) release the superficial layer.

13. The apparatus according to any of the preceding claims wherein the injection device comprises a needle.

14. The apparatus according to claim 13 wherein the needle is inserted into the eye after the second portion has displaced the superficial layer.

15. A conjunctiva displacement device comprising :
a first portion adapted to engage an injection device and moveable in a first plane; and
a second portion coupled to the first portion, wherein the second portion is adapted to displace a superficial layer of an eye in a second plane based on movement of the first portion in the first plane.
